# EUROPEAN PATENT SPECIFICATION

(11) **EP 4 289 389 B1**
(45) Date of publication and mention of the grant of the patent: **11.02.2026**
(21) Application number: 22187019.9
(22) Date of filing: 26.07.2022
(51) Int. Cl.: A61C 8/00

(54) **IMPLANT**
IMPLANTAT
IMPLANT

(30) Priority: 06.06.2022 TW 111120886
(43) Date of publication of application: 13.12.2023
(60) Divisional application: 23178047.9
(73) Proprietor: Sentronic International Corp., New Taipei City (TW)
(72) Inventor: TSAI, Tung-Kuo, New Taipei City (TW)
(74) Representative: Melchior, Robin

(56) References cited:
- WO-A1-2020/222035
- KR-B1- 102 161 197
- US-A- 4 729 766
- US-A1- 2008 145 400
- US-A1- 2010 173 264

## Description

### BACKGROUND

### 1. Field of the Invention

The present invention relates to an implant that is configured to be put into a human body, especially to an implant with surface treatment.

### 2. Description of the Prior Arts

In many surgeries, artificial objects may be put into human bodies, and these artificial objects are socalled implants. One of the common implants is an artificial tooth root for the dental implant surgery. The artificial tooth root is configured to be inserted into the gum, so the gum should be drilled and thus form a hole for the artificial tooth root. After a period of time, the bone of the gum may grow and thus the artificial tooth root can be secured on the gum. However, the growth speed of the bone is very slow, which causes inconvenience to the patient after the surgery. Thus, how to improve the growth speed of the bone is an issue of the dental implant surgery.

In addition, conventional implants are disclosed by US 4,729,766 A1, WO 2020/222035 A1, US 2008/0145400 A1, and KR 10-2161197 B1.

### SUMMARY OF THE INVENTION

State of the art is further exemplified by US 2010/173264 A1 which discloses a dental implant with a bone growth enhancing material in the surface of the implant. The invention is as defined in the independent claim. To overcome the shortcomings, the present invention provides an implant to mitigate or obviate the aforementioned problems.

The main objective of the present invention is to provide a medicine and an implant that is capable of absorbing more medicine, such that the medicine can assist the implanted tissue of the human body in recovery, or prevent the implanted tissue from development.

The implant has an outer surface, a plurality of cavities, and a plurality of passages. The outer surface forms a plurality of openings. The cavities communicates with the openings respectively. A longitudinal section of each one of the cavities is round, oval, or triangular. The longitudinal sections of the cavities are perpendicular to the outer surface. The surface of each one of the cavities comprises irregular concave and convex structures. One end of each one of the passages communicates with a respective one of the openings, and another end of each one of the passages communicates with a respective one of the cavities. A maximum area of a latitudinal section of each one of the cavities is larger than a sectional area of the corresponding opening. The latitudinal sections thereof are parallel with the outer surface. The medicine being configured to be contained in the cavities (12) after flowing into the cavities (12) when applied to the surface of the implant and not to flow out of the cavities.

Therefore, because the outer surface of the implant of the present invention forms multiple openings and the openings communicate with the cavities in the implant, the medicine may flow into the cavities after applied on the implant. The latitudinal section of each cavity is larger than the corresponding opening, so after flowing into the cavities, the medicine would not easily flow out, which allows the implant to absorb more medicine.

Other objectives, advantages and novel features of the invention will become more apparent from the following detailed description when taken in conjunction with the accompanying drawings.

### BRIEF DESCRIPTION OF THE DRAWINGS

Fig. 1 is a perspective view of an implant in accordance with a first embodiment of the present invention;
Fig. 2 is a sectional view of the implant of the first embodiment in Fig. 1;
Fig. 3 is a sectional view of the implant in another configuration of the first embodiment in Fig. 1;
Fig. 4 is a sectional view of the implant in another configuration of the first embodiment in Fig. 1;
Fig. 5 is a sectional view of the implant in another configuration of the first embodiment in Fig. 1.

### DETAILED DESCRIPTION OF THE INVENTION

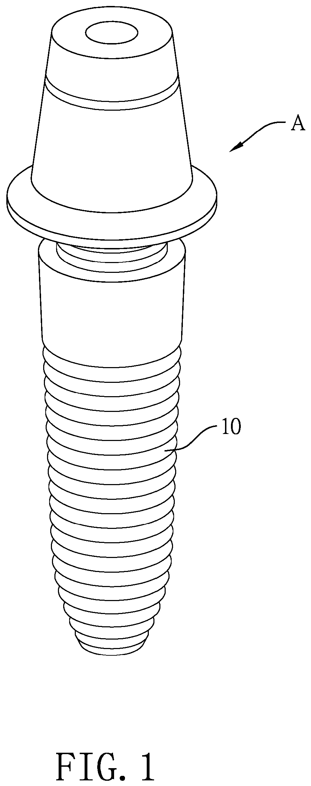

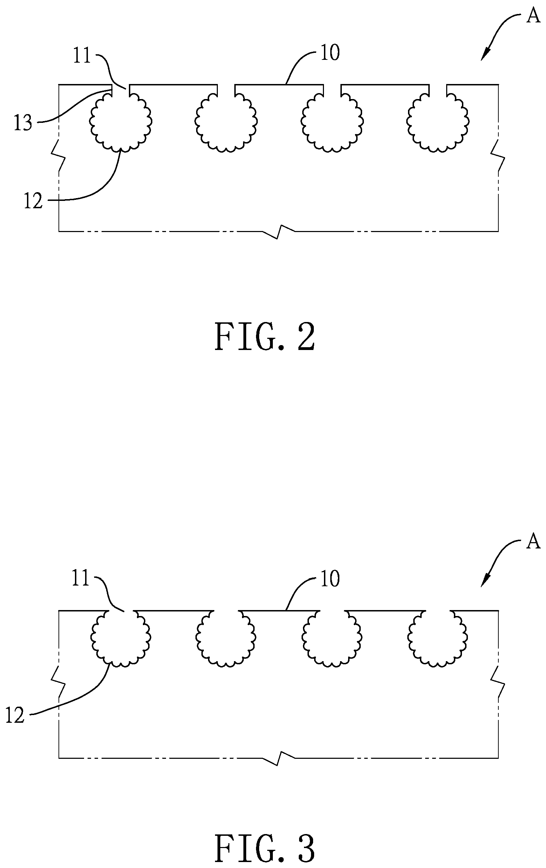

With reference to Fig.1 and Fig. 2, an implant in accordance with the present invention is provided. In a first embodiment, the implant is an artificial tooth root A for the dental implant surgery and comprises an outer surface 10, a plurality of cavities 12 and a plurality of passages 13.

The outer surface 10 forms a plurality of openings 11. The cavities 12 respectively communicate with the openings 11. Each one of the cavities 12 communicates with the corresponding opening 11 via a respective one of the passages 13. Precisely, one end of each passage 13 communicates with one of the openings 11 and another end of said passage 13 communicates with one of the cavities 12. In this embodiment, each opening 11 communicates with one passage 13 and one cavity 12; in another embodiment, one opening 11 may communicate with one cavity 12 only. Fig. 2 shows the arrangement of the cavities 12. In Fig. 2, the openings 11 and the cavities 12 are arranged regularly on the outer surface 10; in another embodiment, the openings 11 and the cavities 12 may be arranged irregularly as shown in Fig. 3.

The longitudinal section of each one of the cavities 12 is perpendicular to the outer surface 10 and may be round, elliptical, oval, or triangular. The latitudinal section of each one of the cavities 12 is parallel with the outer surface 10, and the latitudinal section thereof is changed according to the distance from the outer surface. The maximum area of the latitudinal section is larger than the sectional area of the communicating opening 11. Therefore, the cavities 12 have more spaces to contain medicine. Further, the surface of each one of the cavities 12 may be smooth, or comprise irregular concave and convex structures.

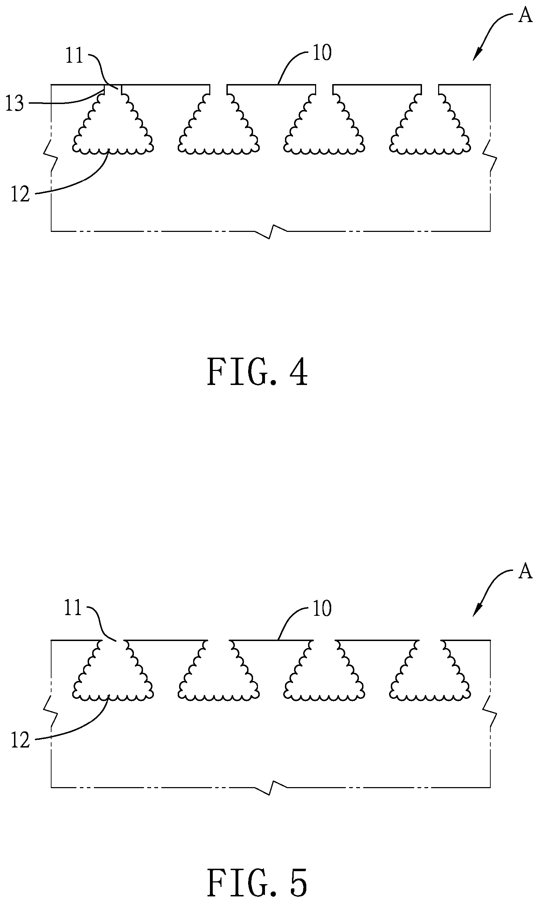

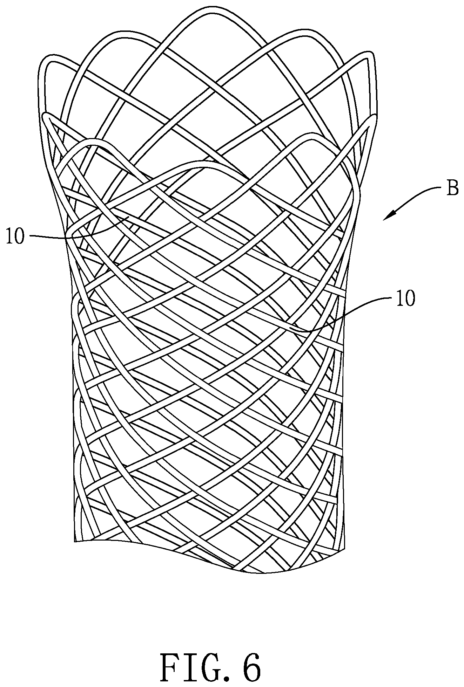

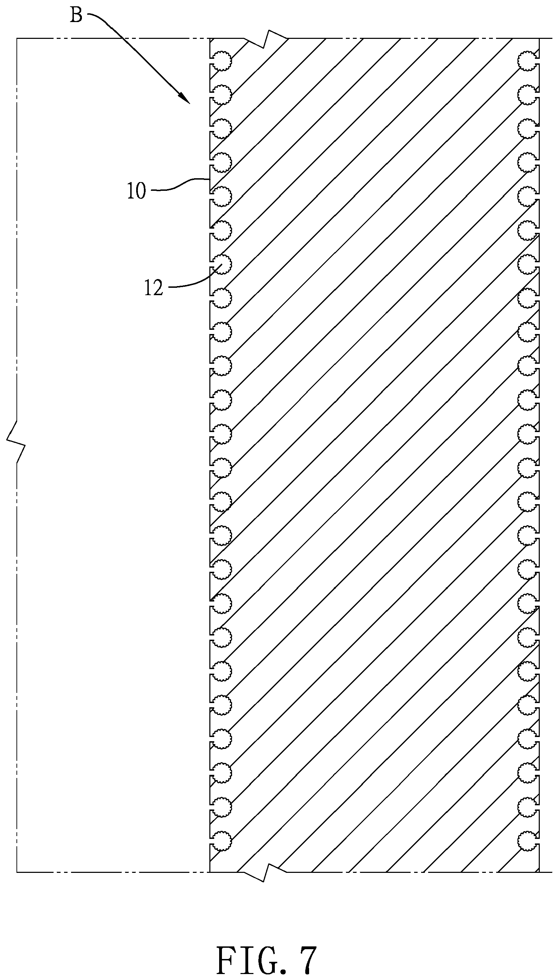

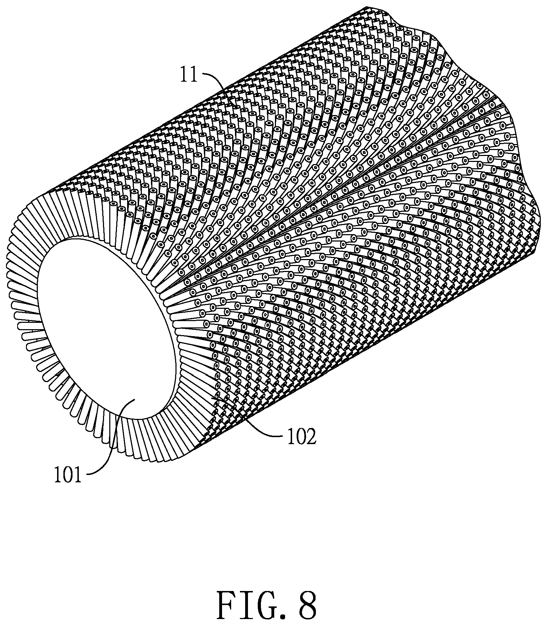

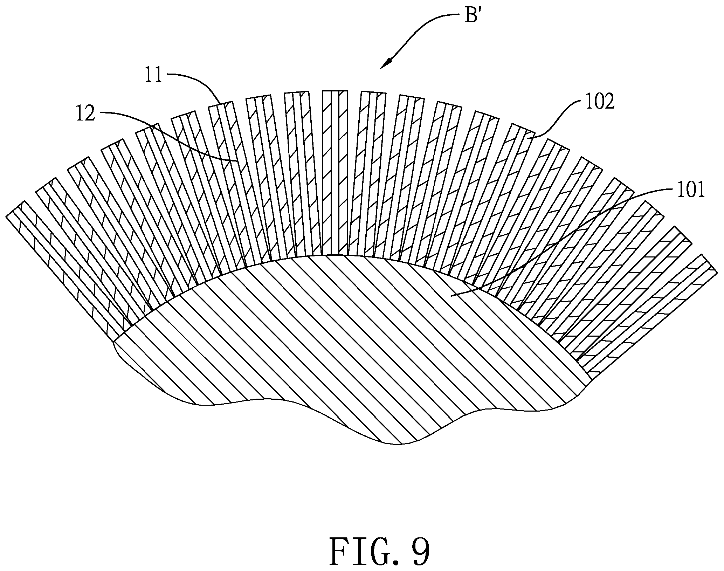

In the configurations as shown in Fig. 4 and Fig. 5, the latitudinal section of each one of the cavities 12 is as larger as farer from the outer surface 10. In other words, in these configurations, the longitudinal sections of the cavities 12 may be triangular.

With the aforesaid structure, the outer surface of the implant forms multiple openings 11 communicating with the cavities 12, and therefore the medicine can flow into the cavities 12 after the medicine is applied on the surface of the implant. Because the maximum of the latitudinal section of each cavity 12 is larger than the corresponding opening 11, the medicine may not flow out after flowing into the cavity 12, which allows the implant to absorb more medicine. If the implant is an artificial tooth root A, the medicine may include ingredients for enhancing the gum bone growth. Therefore, the implant of the present invention can absorb more medicine that enhances bone growth, which shortens the period for fixing the artificial tooth root A on the gum. Therefore, the implant of the present invention can absorb more medicine that prevents angiogenesis, which decreases vascular blockage possibility.

## Claims

1. An implant and a medicine, the implant comprising:
an outer surface (10) forming a plurality of openings (11); and
a plurality of cavities (12) communicating with the openings (11) respectively; a longitudinal section of each one of the cavities (12) being round, oval, or triangular; the longitudinal sections of the cavities (12) being perpendicular to the outer surface (10); a surface of each one of the cavities (12) comprising irregular concave and convex structures;
wherein
the implant comprises:
a plurality of passages (13), one end of each one of the passages (13) communicating with a respective one of the openings (11), and another end of each one of the passages (13) communicating with a respective one of the cavities (12);
a maximum area of a latitudinal section of each one of the cavities (12) is larger than a sectional area of the corresponding opening (11); and
the latitudinal sections thereof are parallel with the outer surface (10);
**characterized in that** the medicine is configured to be contained in the cavities (12) after flowing into the cavities (12)when applied to the surface of the implant and not to flow out of the cavities (12).

2. The implant as claimed in claim 1, wherein the latitudinal section of each one of the cavities (12) is as larger as farer from the outer surface (10); the latitudinal sections of the cavities (12) are parallel with the outer surface (10).

3. The implant as claimed in claim 1 or 2, wherein the implant is an artificial tooth root (A).

## Patentansprüche

1. Implantat und ein Arzneimittel, wobei das Implantat umfasst:
eine Außenfläche (10), die eine Vielzahl von Öffnungen (11) bildet; und
eine Vielzahl von Hohlräumen (12), die jeweils mit den Öffnungen (11) in Verbindung stehen; wobei ein Längsschnitt von jedem der Hohlräume (12) rund, oval oder dreieckig ist; die Längsschnitte der Hohlräume (12) senkrecht zur Außenfläche (10) stehen; eine Oberfläche von jedem der Hohlräume (12) unregelmäßige konkave und konvexe Strukturen umfasst;
wobei das Implantat umfasst:
eine Vielzahl von Durchgängen (13), wobei ein Ende von jedem der Durchgänge (13) mit einer jeweiligen der Öffnungen (11) in Verbindung steht, und ein anderes Ende von jedem der Durchgänge (13) mit einem jeweiligen der Hohlräume (12) in Verbindung steht;
eine maximale Fläche eines Querschnitts von jedem der Hohlräume (12) größer ist als eine Querschnittsfläche der entsprechenden Öffnung (11); und
wobei die Querschnitte davon parallel zur Außenfläche (10) sind;
**dadurch gekennzeichnet, dass** das Arzneimittel so konfiguriert ist, dass es nach dem Einfließen in die Hohlräume (12) beim Aufbringen auf die Oberfläche des Implantats in den Hohlräumen (12) verbleibt und nicht aus den Hohlräumen (12) herausfließt.

2. Implantat nach Anspruch 1, wobei der Querschnitt jedes der Hohlräume (12) mit zunehmendem Abstand von der Außenfläche (10) größer wird; wobei die Querschnitte der Hohlräume (12) parallel zur Außenfläche (10) sind.

3. Implantat nach Anspruch 1 oder 2, wobei das Implantat eine künstliche Zahnwurzel (A) ist.

## Revendications

1. Implant et médicament, l'implant comprenant:
une surface extérieure (10) formant une pluralité d'ouvertures (11); et
une pluralité de cavités (12) communiquant respectivement avec les ouvertures (11); une section longitudinale de chacune des cavités (12) étant ronde, ovale ou triangulaire; les sections longitudinales des cavités (12) étant perpendiculaires à la surface extérieure (10); une surface de chacune des cavités (12) comprenant des structures concaves et convexes irrégulières;
dans lequel l'implant comprend:
une pluralité de passages (13), une extrémité de chacun des passages (13) communiquant avec une ouverture (11) correspondante, et une autre extrémité de chacun des passages (13) communiquant avec une cavité (12) correspondante;
une aire maximale d'une section latitudinale de chacune des cavités (12) est supérieure à une aire de section de l'ouverture (11) correspondante; et
les sections latitudinales correspondantes sont parallèles à la surface extérieure (10);
**caractérisé en ce que** le médicament est configuré pour être contenu dans les cavités (12) après avoir pénétré dans les cavités (12) lorsqu'il est appliqué sur la surface de l'implant et pour ne pas s'écouler hors des cavités (12).

2. Implant selon la revendication 1, dans lequel la section latitudinale de chacune des cavités (12) est d'autant plus grande qu'elle est éloignée de la surface extérieure (10) ; les sections latitudinales des cavités (12) étant parallèles à la surface extérieure (10).

3. Implant selon la revendication 1 ou 2, dans lequel l'implant est une racine de dent artificielle (A).
